(19) Europäisches Patentamt
European Patent Office
Office européen des brevets

(11) **EP 4 245 750 A1**

(12) **EUROPEAN PATENT APPLICATION**
published in accordance with Art. 153(4) EPC

(43) Date of publication:
**20.09.2023 Bulletin 2023/38**

(21) Application number: **21891014.9**

(22) Date of filing: **02.11.2021**

(51) International Patent Classification (IPC):
*C07C 237/08* (2006.01) *C07C 231/12* (2006.01)
*C07C 231/02* (2006.01) *C07C 233/25* (2006.01)
*C07C 213/02* (2006.01) *C07C 217/02* (2006.01)
*C07C 201/12* (2006.01) *C07C 205/37* (2006.01)
*C07C 41/22* (2006.01) *C07C 43/225* (2006.01)
*C07F 9/54* (2006.01) *A61K 31/661* (2006.01)
*A61K 31/167* (2006.01) *A61P 35/00* (2006.01)
*A61P 35/02* (2006.01)

(86) International application number:
**PCT/CN2021/128275**

(87) International publication number:
**WO 2022/100487 (19.05.2022 Gazette 2022/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.11.2020 CN 202011271276**

(71) Applicant: **YIWUHUAYAO PHARMACEUTICAL TECHNOLOGY CO., LTD**
**Yiwu City Jinhua, Zhejiang 322000 (CN)**

(72) Inventor: **WANG, Jianping**
**Jinhua, Zhejiang 322000 (CN)**

(74) Representative: **Cabinet Chaillot**
**16/20, avenue de l'Agent Sarre**
**B.P. 74**
**92703 Colombes Cedex (FR)**

(54) **AMINO-COMBRETASTATIN DERIVATIVE AND USE THEREOF**

(57) Disclosed is an amino-combretastatin derivative, and in particular a compound represented by general formula (I), a pharmaceutically acceptable salt thereof, a hydrate thereof, or a hydrate of the pharmaceutically acceptable salt thereof, a solvate thereof or a solvate of the pharmaceutically acceptable salt thereof, and an isomer thereof. In general formula (I), $R^1$ is selected from a C1-C3 alkyl or a C1-C3 haloalkyl, and $R^2$ and $R^3$ are respectively and independently selected from a C1-C6 alkyl, a C1-C6 haloalkyl or a C1-C6 alcohol group. The present invention also relates to the use of such compounds in the preparation of drugs for the treatment of diseases caused by abnormal neovascularization, and tubulin aggregation inhibitors.

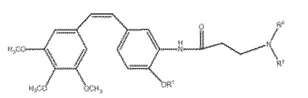

EP 4 245 750 A1

## Description

### TECHNICAL FIELD

The present invention relates to the field of pharmaceutical compound synthesis and preparation, particularly to the synthesis and preparation of anticancer pharmaceutical compounds, and more particularly to amino-combretastatin derivatives, synthesis methods and use thereof.

### BACKGROUND

Combretastatin-based compounds are a series of compounds extracted and isolated from the trunk of Combrettumcaffrum in South Africa having a cis-1,2-diphenylethene structure. Among these, combretastatin A-4, or CA-4 (*cis*-1-(3,4,5-trimethoxy)phenyl-2-(3'-hydroxy-4'-methoxy)phenylethylene), has the strongest effect of inhibiting microtubule aggregation. In 1997, Hatanaka et al. of Ajinomoto Co., Ltd. found that the anticancer activity can be greatly improved by changing the hydroxyl group at the 3' position of CA-4 to an amino group. However, the poor water solubility of combretastatin-based compounds is further reduced after changing the 3' position of CA-4 into an amino group. Ajinomoto Co., Ltd. modified the amino-CA-4 with amino acids to increase its water solubility, and formulated it into injections for cancer patients. However, studies showed that the toxicity of amino-CA-4 greatly ascends after modification with amino acids, resulting in decreased tolerability in humans and thus unsuitability for clinical use.

Therefore, further modifying the structure of amino-combretastatin, so as to reduce toxicity, improve tolerance and ensure the therapeutic effect on the premise of meeting the water-solubility requirements of different formulations, particularly oral formulations, is a key problem of interest and an urgent technical problem for technicians in the art, particularly in the art of combretastatin-based compounds.

### SUMMARY

The technical problem to be solved by the present invention is how to improve the water solubility of the amino-combretastatin while ensuring good and clinically acceptable toxicity and tolerability.

In order to solve the above technical problems, the present invention provides a compound of general formula (I), a pharmaceutically acceptable salt thereof, a hydrate thereof, a solvate thereof, a solvate of the pharmaceutically acceptable salt and an isomer thereof, wherein the general formula (I) is:

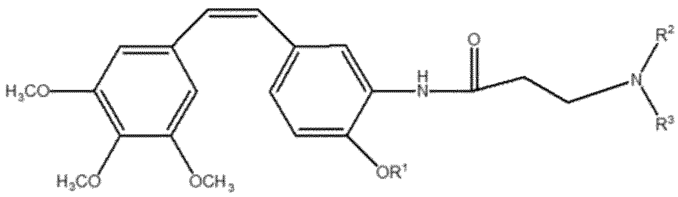

wherein $R^1$ is selected from a C1-C3 alkyl and a C1-C3 haloalkyl;

$R^2$ and $R^3$ are each independently selected from a C1-C6 alkyl, a C1-C6 haloalkyl and a C1-C6 alcohol group.

More preferably, $R^1$ is selected from methyl, ethyl, difluoromethyl and trifluoroethyl.

In a preferred technical scheme, $R^2$ and $R^3$ are each independently selected from methyl, ethyl, *n*-propyl, isopropyl, *n*-butyl, *n*-pentyl, *n*-hexyl, propenyl and propynyl.

In a preferred technical scheme, $R^2$ and $R^3$ are both methyl, ethyl, *n*-propyl, isopropyl, *n*-butyl, *n*-pentyl, *n*-hexyl, propenyl or propynyl.

More preferably, the compound disclosed herein is preferably (*Z*)-1-(3,4,5-trimethoxyphenyl)-2-((3-(bis(2-*n*-propyl)amino)propionamide)-4-ethoxyphenyl)e thylene,

(*Z*)-1-(3,4,5-trimethoxyphenyl)-2-((3-(bis(2-*n*-propyl)amino)propionamide)-4-methoxyphenyl )ethylene,
(*Z*)-1-(3,4,5-trimethoxyphenyl)-2-((3-(bis(2-*n*-butyl)amino)propionamide)-4-ethoxyphenyl)et hylene,
(*Z*)-1-(3,4,5-trimethoxyphenyl)-2-((3-(bis(2-*n*-butyl)amino)propionamide)-4-methoxyphenyl) ethylene,
(*Z*)-1-(3,4,5-trimethoxyphenyl)-2-((3-(bis(2-ethyl)amino)propionamide)-4-ethoxyphenyl)ethy lene,
(*Z*)-1-(3,4,5-trimethoxyphenyl)-2-((3-(bis(2-*n*-propyl)amino)propionamide)-4-trifluoroethoxy phenyl),
(*Z*)-1-(3,4,5-trimethoxyphenyl)-2-((3-(bis(2-*n*-propyl)amino)propionamide)-4-difluoromethox yphenyl)ethylene,

(*Z*)-1-(3,4,5-trimethoxyphenyl)-2-((3-(bis(2-*n*-butyl)amino)propionamide)-4-trifluoroethoxyp henyl)ethylene,
(*Z*)-1-(3,4,5-trimethoxyphenyl)-2-((3-(bis(2-*n*-butyl)amino)propionamide)-4-difluoromethoxy phenyl)ethylene,
(*Z*)-1-(3,4,5-trimethoxyphenyl)-2-((3-(bis(2-hydroxyethyl)amino)propionamide)-4-ethoxyphe nyl)ethylene,
(*Z*)-1-(3,4,5-trimethoxyphenyl)-2-((3-(bis(2-hydroxyethyl)amino)propionamide)-4-methoxyp henyl)ethylene,
(*Z*)-1-(3,4,5-trimethoxyphenyl)-2-((3-(2,2-difluoroethylamino)propionamide)-4-ethoxyphenyl )ethylene or
(*Z*)-1-(3,4,5-trimethoxyphenyl)-2-((3-(2,2-dimethoxyethylamino)propionamide)-4-ethoxyphe nyl)ethylene.

The pharmaceutically acceptable salt described herein refers to a salt formed by one or more basic salt-forming groups and an acid. The acid may be an organic acid or an inorganic acid. Among these, the preferable scheme is to form the salt with one or more of hydrochloric acid, sulfuric acid, phosphoric acid, acetic acid, propionic acid, trifluoroacetic acid, glycolic acid, succinic acid, maleic acid, fumaric acid, malic acid, tartaric acid, citric acid, oxalic acid, amino acid, benzoic acid, salicylic acid, 4-aminosalicylic acid, mandelic acid, cinnamic acid, nicotinic acid, isonicotinic acid, methanesulfonic acid, trifluoromethanesulfonic acid, ethanesulfonic acid, 2-hydroxyethanesulfonic acid, benzenesulfonic acid, p-toluenesulfonic acid and 2-naphthalenesulfonic acid. A more preferable technical scheme is that the pharmaceutically acceptable salt is a hydrochloride, a sulfate, a phosphate, a citrate or a tartrate.

It is noted that the solvate used described herein includes an organic solvate and an inorganic solvate. The present invention comprises different physical forms of the compound of general formula (I) and the pharmaceutically acceptable salt thereof, such as solvate, hydrate, non-solvate or non-hydrate.

The isomer may be in a mixture or an isolated form. As used herein, the isomer includes both stereoisomers in the form of a mixture and stereoisomers in an isolated form, i.e., all possible stereoisomers and mixtures thereof are included. More preferably, the isomer used herein refers to optical isomers having a specific activity, generally optical isomers in the form of racemic isomers which can be separated.

Meanwhile, the present invention further discloses a synthesis method of the compound of general formula (I) through the following reactions:

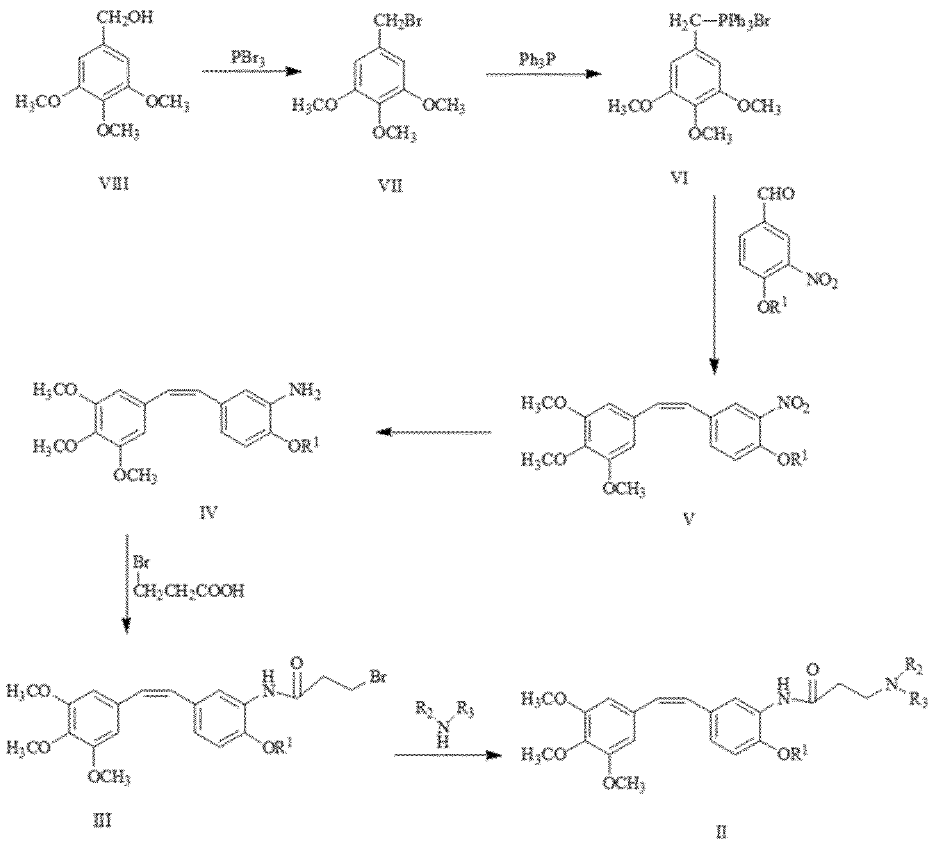

VIII VII VI V IV III II

The synthesis method disclosed herein obtains VI through bromination and reaction with triphenylphosphine, and then obtains *cis* compound V through Wittig reaction. The nitro group in compound V is then reduced to an amino group by using reductants dioctyl pyridinium dibromide and samarium powder to obtain amino-combretastatin A-4. Amino-combretastatin A-4, as the starting material, is then esterified with 2-bromopropionic acid under the catalysis of DMTMM to obtain compound III. Compound III and an amine compound react at a high temperature to obtain compound II.

More preferably, compound II is further salified with an acid to form a pharmaceutically acceptable salt product.

More preferably, the method further comprises separating optical isomers, wherein the optical isomers of compound II are resolved by a physical method or in the salification step. The physical method may be a known method for separating optical isomers, such as fractional crystallization, separation of diastereomeric derivatives or separation by chiral chromatography. Separation in the salification step may be an existing separation method through salification, such as through salification with an optically active acid and crystallization to obtain isolated optical isomers from a racemate.

The present invention further provides a medicament or pharmaceutical composition prepared from the compound of general formula (I), the pharmaceutically acceptable salt thereof, the hydrate thereof, the solvate thereof, the solvate of the pharmaceutically acceptable salt or the isomer thereof.

Preferably, the medicament or pharmaceutical composition is in a formulation for topical, enteral or parenteral administration.

When prepared into a formulation, the medicament or pharmaceutical composition may be inorganic or organic, and may be in a solid or liquid state. For example, when used for oral administration, the medicament or pharmaceutical composition can be prepared into conventional solid formulations such as tablets, capsules, pills and granules, and can also be prepared into common liquid formulations such as oral liquids, oral suspensions, syrups and the like. For another example, where forms for parenteral administration or injections are applicable, isotonic aqueous solutions or emulsions are preferred. In the case of a lyophilized composition consisting exclusively of the active ingredient and one carrier, such solutions may be prepared prior to use. These pharmaceutical compositions may be sterile, or comprise an excipient, or a solvent and an osmoregulatory salt.

Preferably, the present invention discloses a tablet or a capsule prepared from the compound of general formula (I), the pharmaceutically acceptable salt thereof, the hydrate thereof, the solvate thereof, the solvate of the pharmaceutically acceptable salt or the isomer thereof.

When a tablet or capsule is prepared, the tablet or capsule comprises the active ingredient, a diluent (e.g., lactose, glucose, sucrose, mannitol, sorbitol, cellulose, glycerol), a lubricant (e.g., talc, a stearate) and polyethylene glycol. The tablet comprises a binder, starch, gelatin, methylcellulose, carboxymethylcellulose and polyvinylpyrrolidone, and, if desired, may further comprise a disintegrant (such as starch, agar, alginic acid and salts thereof), an effervescent mixture or an adsorbent, a dye, a flavoring agent or a sweetener.

The present invention further discloses use of the compound of general formula (I), the pharmaceutically acceptable salt thereof, the hydrate thereof, the solvate thereof, the solvate of the pharmaceutically acceptable salt and the isomer thereof in preparing a medicament for treating a disease caused by abnormal neovascularization.

Diseases with evident cause of abnormal neovascularization include various tumors such as lung cancer, small cell lung cancer, liver cancer, pancreatic cancer, gastric cancer, bone cancer, esophageal cancer, breast cancer, kidney cancer, bile duct cancer, prostate cancer, testicular cancer, colon cancer, bladder cancer, cervical cancer, bronchial cancer, melanoma, adenocarcinoma, hidradhoma carcinoma, papillary carcinoma, papillary adenocarcinoma, squamous cell carcinoma, basal cell carcinoma, cystic adenocarcinoma, glioma, astrocytoma, neuroblastoma, medulloblastoma, craniopharyngioma, ependymoma, pinealoma, hemangioblastoma, oligodendroglioma, meningioma, neurofibroma, fibrosarcoma, fibroblastoma, fibroma, myxosarcoma, myxocystoma, lipoma, lipoadenoma, chondrosarcoma, chondroma, chondromyoma, chordoma, chorioadenoma, chorioangioma, chorioepithelioma, chorioblastoma, osteosarcoma, osteoblastoma, osteoclastoma, osteochondrofibroma, osteochondrosarcoma, femorale cystoma, cementoblastoma, osteofibroma, osteofibrosarcoma, hemangioma, angiosarcoma, lymphangiosarcoma, lymphangioma, lymphoma, endothelioma, synovioma, synoviosarcoma, mesothelioma, mesocytoma, Ewing's sarcoma, leiomyoma, rhabdomyoma, rhabdomyosarcoma, acute lymphocytic leukemia, acute myeloid leukemia, chronic leukemia, polycythemia and multiple myeloma, and rheumarthritis, diabetic retinopathy, retinopathy of prematurity, retinal vein occlusion, psoriasis, rosacea, Kaposi's sarcoma, specific reactive keratitis, epidemic keratoconjunctivitis, neovascular glaucoma, bacterial ulcer, fungal ulcer, ulcerative colitis, chronic granulomatous, chronic granulomatosis, granulomato, herpes simplex infection, herpes zoster infection, protozoan infection, mycobacteria infection, polyarteritis, sarcoid, scleritis, flushing, xerostomia, arthritis syndrome, systemic lupus erythematosus, AIDS syndrome, syphilis, etc.

Meanwhile, the present invention further discloses use of the compound of general formula (I), the pharmaceutically acceptable salt thereof, the hydrate thereof, the solvate thereof, the solvate of the pharmaceutically acceptable salt and the isomer thereof in preparing a tubulin aggregation inhibitor.

## DETAILED DESCRIPTION

Exemplary embodiments of the present invention will be described in detail below. These embodiments are for illustrative purposes only and are not intended to limit the scope of the present invention.

The followings are definitions of terms used herein.

Unless otherwise specified, the initial definition provided herein with respect to a group or term applies to that

group or term throughout the specification, whether used alone or as part of another group.

The term "alkyl" refers to an unsubstituted linear or branched hydrocarbon group having 1 to 20 carbon atoms, preferably 1 to 6 carbon atoms, and particularly, methyl, ethyl, propyl (n-propyl and isopropyl), butyl (*n*-butyl, isobutyl and *tert*-butyl) and the like.

The term "alkenyl" refers to an alkene having one or more carbon-carbon double bonds, such as ethenyl, propenyl, 1,3-butadiene, *cis*-butene, *trans*-butene and the like.

The term "alkynyl" refers to an alkyne having one or more carbon-carbon triple bonds, such as ethynyl, propynyl and the like.

The term "halogen" or "halo" refers to fluorine, chlorine, bromine or iodine.

The term hydroxy refers to the group -OH.

The term carboxyl refers to the group -COOH.

The term amino refers to the group $-NH_2$.

The term nitro refers to the group $-NO_2$.

The term alkoxy refers to the group $-OR^4$, wherein $R^4$ refers to an alkyl.

The term carbamoyl refers to the group $R^5C(=O)NH_2$, wherein $R^5$ refers to an alkyl.

The term cyano refers to the group -CN.

The term amido refers to the group -C(=O)NH-.

The term alkoxycarbonyl refers to the group $-C(=O)OR^6$, wherein $R^6$ refers to an alkyl.

The term acyloxy refers to the group $-OC(=O)R^7$, wherein $R^7$ refers to an alkyl.

The term sulfhydryl refers to the group -SH.

"Substituted" means that the subsequent groups may be substituted with some common groups (e.g., hydrogen, halogen, hydroxy, amino, sulfhydryl, nitro, cyano, aryl, heterocyclyl, heterocycloalkyl, carboxy, amido, etc.).

"Optional" means that the subsequent event or circumstance may, but not necessarily, occur. The description includes instances where the event or circumstance occurs and instances where it does not.

As used herein, "pharmaceutically acceptable carrier" includes all solvents, dispersion media, coatings, antibacterials and antifungals, isotonic and absorption delaying agents, and the like. Such media and agents for pharmaceutically active substances are well known in the art. Except for any conventional media or agents incompatible with the active ingredient, the use of the media or agents in the therapeutic composition is contemplated. Additional active ingredients may also be incorporated into the composition.

**Example 1**

Synthesis of (*Z*)-1-(3,4,5-trimethoxyphenyl)-2-(3-(bis(2-*n*-propyl)amino)propionamide)-4-ethoxyphenyl)ethylene

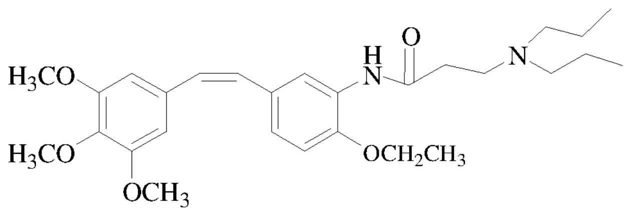

Step 1: Synthesis of trimethoxybenzyl triphenylphosphine bromide

320 g of 3,4,5-trimethoxybenzyl alcohol was dissolved in 2 L of toluene. The mixture was cooled to -5 to 0 °C after stirring and dissolving. 100 mL of phosphorus bromide was dropwise added with a constant-pressure dropping funnel with the reaction temperature kept at -5 to 0 °C. After the dropwise addition, the system was kept at a low temperature for 2 h of reaction and warmed to room temperature for reaction overnight. 1.4 L of purified water was added to quench the reaction. The mixture was stirred for 30 min, and let stand for separating the phases. The upper organic phase was washed with saturated sodium bicarbonate solution to pH 7.5 to 8, dried over anhydrous magnesium sulfate and filtered to give a solution of trimethoxybenzyl bromide in toluene. 0.56 kg of triphenylphosphine was added into the solution. The system was stirred for more than 48 h until a solid was precipitated, and was filtered to obtain a crude product. The crude product was recrystallized in absolute ethanol.

Step 2: Synthesis of (Z)-1-(3,4,5-trimethoxyphenyl)-2-(3-amino-4-ethoxyphenyl)ethylene

In a reactor with an argon atmosphere, 15 g of trimethoxyphenyl methylene triphenylphosphine bromide was

dissolved in 300 mL of anhydrous THF. The solution was cooled to about -15 °C before 22 mL of a 1.6 mol/L solution of n-butyllithium in cyclohexane was dropwise added, and the system was reacted for 1 h. A solution of 3-nitro-4-ethoxybenzaldehyde (5.7 g) in THF (24 mL) was slowly added dropwise to the reaction system. The reaction system was gradually warmed to room temperature, stirred overnight and monitored by TLC. After the reaction was completed, the system was cooled to -5 °C, and saturated brine was added to quench the reaction. The phases were separated, and the organic phase was concentrated and separated with a silica gel column (n-hexane:ethyl acetate = 4:1) to obtain 6.5 g of (Z)-1-(3,4,5-trimethoxyphenyl)-2-(3-nitro-4-ethoxyphenyl)ethylene as a light yellow crystal (50% to 60% yield).

In a dry 1-L four-necked flask with an argon atmosphere, 700 mL of anhydrous isopropanol was added, followed by carefully adding 10 g of dioctyl pyridinium dibromide and 24 g of samarium powder. Finally, 10 g of (Z)-1-(3,4,5-trimethoxyphenyl)-2-(3-nitro-4-ethoxyphenyl)ethylene was added. The system was stirred, heated at reflux and monitored by TLC until the reaction was completed. After the reaction was completed, the mixture was filtered at reduced pressure, and the filtrate was concentrated and subjected to silica gel column chromatography (petroleum ether:ethyl acetate = 4:1). The yield was 30% to 40%.

Step 3: Synthesis of (Z)-1-(3,4,5-trimethoxyphenyl)-2-(3-bromopropionamide)-4-ethoxyphenyl)ethylene

10 g of (Z)-1-(3,4,5-trimethoxyphenyl)-2-(3-amino-4-ethoxyphenyl)ethylene was dissolved in absolute ethanol before 20 g of DMTMM was added. After complete dissolution, 5.58 g of bromopropionic acid was added. The reaction was conducted at room temperature and monitored by TLC. After the reaction was completed, the mixture was quickly concentrated at a low temperature, dissolved in ethyl acetate and washed with water. After washing, the organic phases were combined and concentrated. The residual was recrystallized in ethyl acetate:petroleum ether = 1:4 to obtain a white or yellowish solid (60% to 70% yield).

Step 4: Synthesis of (*Z*)-1-(3,4,5-trimethoxyphenyl)-2-((3-(bis(2-*n*-propyl)amino)propionamide)-4-ethoxyphenyl)e thylene

2 g of (*Z*)-1-(3,4,5-trimethoxyphenyl)-2-(3-bromopropionamide)-4-ethoxyphenyl)ethylene was dissolved in 10 mL of ethanol before 0.53 g of dipropylamine and 2 mL of triethylamine were added. The mixture was then heated to 60 °C and stirred for reaction. The reaction was monitored by TLC. After the reaction was completed, the mixture was concentrated and subjected to silica gel column chromatography (ethyl acetate:petroleum ether = 1:6) at normal pressure. After concentration, 1.65 g of (*Z*)-1-(3,4,5-trimethoxyphenyl)-2-((3-(bis(2-*n*-propyl)amino)propionamide)-4-ethoxyphenyl)e thylene, as a white crystal, was obtained (about 80% yield). MS(m/Z)=484.30. $^1$HNMR(ppm)$\delta$: 7.08(d,1H,2'-H);6.92(d,1H,6'-H);6.76(d,1H,5'-H);6.62(s,2H,2,6'-H);6.49(d,1H,J=12.2Hz,1aH);6.43(d,1H,J=12.2Hz,1a'-H);4.18(q,2H,-$CH_2$);3.86(s,3H,4-$OCH_3$);3.70(s,6H,3,5-$OCH_3$);2. 74(t,2H,-$CH_2$);2.36(dt,4H,-$NCH_2$-);2.33(t,2H,-$COCH_2$);1.43(m,4H,-$CH_2$-);1.33(t,3H,-$CH_3$);0. 96(t,6H,-$CH_3$).

**Example 2**

Preparation of (*Z*)-1-(3,4,5-trimethoxyphenyl)-2-((3-(bis(2-*n*-propyl)amino)propionamide)-4-ethoxyphenyl)e thylene hydrochloride 1 g of (*Z*)-1-(3,4,5-trimethoxyphenyl)-2-((3-(bis(2-*n*-propyl)amino)propionamide)-4-ethoxyphenyl)e thylene was dissolved in 10 mL of methanol before 0.2 mL of hydrochloric acid was added. The mixture was heated to 35 °C and stirred for 1 h. After cooling, the mixture was concentrated, and 10 mL of water is added. The resultant mixture was stirred for 10 min and let stand overnight. The mixture was filtered at reduced pressure, and the resultant white filter cake was lyophilized to give (*Z*)-1-(3,4,5-trimethoxyphenyl)-2-((3-(bis(2-*n*-propyl)amino)propionamide)-4-ethoxyphenyl)e thylene hydrochloride.

**Example 3**

Preparation of (*Z*)-1-(3,4,5-trimethoxyphenyl)-2-((3-(bis(2-*n*-propyl)amino)propionamide)-4-methoxyphenyl )ethylene

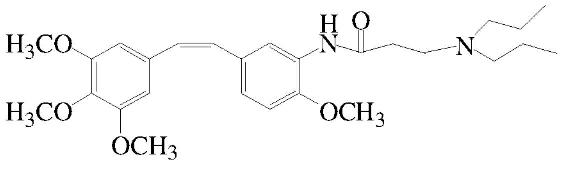

Step 1: Synthesis of trimethoxybenzyl triphenylphosphine bromide

320 g of 3,4,5-trimethoxybenzyl alcohol was dissolved in 2 L of toluene. The mixture was cooled to -5 to 0 °C after stirring and dissolving. 100 mL of phosphorus bromide was dropwise added with a constant-pressure dropping funnel with the reaction temperature kept at -5 to 0 °C. After the dropwise addition, the system was kept at a low temperature for 2 h of reaction and warmed to room temperature for reaction overnight.

1.4 L of purified water was added to quench the reaction. The mixture was stirred for 30 min, and let stand for separating the phases. The upper organic phase was washed with saturated sodium bicarbonate solution to pH 7.5 to 8, dried over anhydrous magnesium sulfate and filtered to give a solution of trimethoxybenzyl bromide in toluene.

0.56 kg of triphenylphosphine was added into the solution. The system was stirred for more than 48 h until a solid was precipitated, and was filtered to obtain a crude product. The crude product was recrystallized in absolute ethanol.

Step 2: Synthesis of (*Z*)-1-(3,4,5-trimethoxyphenyl)-2-(3-amino-4-methoxyphenyl)ethylene In a reactor with an argon atmosphere, 15 g of trimethoxyphenyl methylene triphenylphosphine bromide was dissolved in 300 mL of anhydrous THF. The solution was cooled to about -15 °C before 22 mL of a 1.6 mol/L solution of *n*-butyllithium in cyclohexane was dropwise added, and the system was reacted for 1 h. A solution of 3-nitro-4-methoxybenzaldehyde (5.5 g) in THF (24 mL) was slowly added dropwise to the reaction system. The reaction system was stirred overnight, gradually warmed to room temperature and monitored by TLC. The next day, the reaction was cooled to -5 °C and quenched by addition of saturated brine. The phases were separated, and the organic phase was concentrated and separated with a silica gel column (*n*-hexane:ethyl acetate = 4:1) to obtain 6.0 g of (Z)-1-(3,4,5-trimethoxyphenyl)-2-(3-nitro-4-methoxyphenyl)ethylene as a light yellow crystal (50% to 60% yield).

In a dry 1-L four-necked flask with an argon atmosphere, 700 mL of anhydrous isopropanol was added, followed by carefully adding 10 g of dioctyl pyridinium dibromide and 24 g of samarium powder. Finally, 10 g of (Z)-1-(3,4,5-trimethoxyphenyl)-2-(3-nitro-4-methoxyphenyl)ethylene was added. The system was stirred, heated at reflux and monitored by TLC until the reaction was completed. After the reaction was completed, the mixture was filtered at reduced pressure, and the filtrate was concentrated and subjected to silica gel column chromatography (petroleum ether:ethyl acetate = 4:1). The yield was 30% to 40%.

Step 3: (*Z*)-1-(3,4,5-trimethoxyphenyl)-2-((3-(bis(2-*n*-propyl)amino)propionamide)-4-methoxyphenyl )ethylene was synthesized according to the method in Example 1. MS(m/Z)=470.28. $^1$HNMR(ppm)δ: 7.08(d,1H,2'-H);6.92(d,1H,6'-H);6.76(d,1H,5'-H);6.62(s,2H,2,6'-H);6.49(d,1H,J=12.2Hz,1aH);6.43(d,1H,J=12.2Hz,1a'-H);3.86(s,3H,4-$OCH_3$);3.73(s,3H,-$OCH_3$);3.70(s,6H,3,5-$OCH_3$);2 .74(t,2H,-$CH_2$);2.36(dt,4H,-$NCH_2$-);2.33(t,2H,-$COCH_2$);1.43(m,4H,-$CH_2$-);0.96(t,6H,-$CH_3$).

**Example 4**

Preparation of (*Z*)-1-(3,4,5-trimethoxyphenyl)-2-((3-(bis(2-ethyl)amino)propionamide)-4-ethoxyphenyl)ethy lene

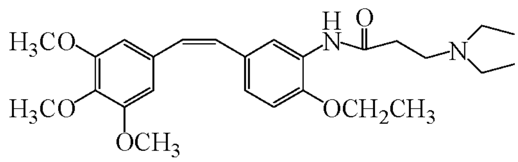

Step 1: (*Z*)-1-(3,4,5-trimethoxyphenyl)-2-(3-bromopropionamide)-4-ethoxyphenyl)ethylene was synthesized according to the method in Example 1.

Step 2: Synthesis of (*Z*)-1-(3,4,5-trimethoxyphenyl)-2-((3-(bis(2-ethyl)amino)propionamide)-4-ethoxyphenyl)ethy lene 2 g of (Z)-1-(3,4,5-trimethoxyphenyl)-2-(3-bromopropionamide)-4-ethoxyphenyl)ethylene was dissolved in 10 mL of ethanol before 0.56 g of diethylamine and 2 mL of triethylamine were added. The mixture was then heated to 60 °C and stirred for reaction. The reaction was monitored by TLC. After the reaction was completed, the mixture was concentrated and subjected to silica gel column chromatography (ethyl acetate:petroleum ether = 1:6) at normal pressure. After concentration, 1.65 g of (*Z*)-1-(3,4,5-trimethoxyphenyl)-2-((3-(bis(2-ethyl)amino)propionamide)-4-ethoxyphenyl)ethy lene, as a white crystal, was obtained (about 80% yield).

MS(m/Z)=456.26. $^1$HNMR(ppm)δ: 7.08(d,1H,2'-H);6.92(d,1H,6'-H);6.76(d,1H,5'-H);6.62(s,2H,2,6'-H);6.49(d,1H,J=12.2Hz,1aH);6.43(d,1H,J=12.2Hz,1a'-H);4.18(q,2H,-$CH_2$);3.86(s,3H,4-$OCH_3$);3.70(s,6H,3,5-$OCH_3$);3. 68(t,2H,-$CH_2$N=);3.01(dt,4H,-$NCH_2$-);2.55(t,2H,-$COCH_2$); 1.33(t,3H,-$CH_3$);1.15(t,6H,-$CH_3$).

### Example 5

Preparation of (*Z*)-1-(3,4,5-trimethoxyphenyl)-2-((3-(bis(2-*n*-butyl)amino)propionamide)-4-ethoxyphenyl)et hylene

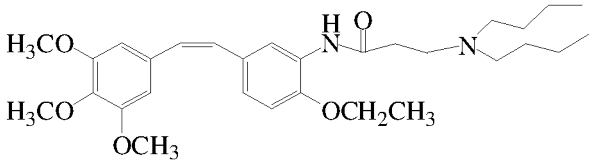

Step 1: (*Z*)-1-(3,4,5-trimethoxyphenyl)-2-(3-bromopropionamide)-4-ethoxyphenyl)ethylene was synthesized according to the method in Example 1.

Step 2: Synthesis of (*Z*)-1-(3,4,5-trimethoxyphenyl)-2-((3-(bis(2-*n*-propyl)amino)propionamide)-4-ethoxyphenyl)e thylene 2 g of (*Z*)-1-(3,4,5-trimethoxyphenyl)-2-(3-bromopropionamide)-4-ethoxyphenyl)ethylene was dissolved in 10 mL of ethanol before 0.56 g of dibutylamine and 2 mL of triethylamine were added. The mixture was then heated to 60 °C and stirred for reaction. The reaction was monitored by TLC. After the reaction was completed, the mixture was concentrated and subjected to silica gel column chromatography (ethyl acetate:petroleum ether = 1:6) at normal pressure. After concentration, 1.65 g of (*Z*)-1-(3,4,5-trimethoxyphenyl)-2-((3-(bis(2-*n*-propyl)amino)propionamide)-4-ethoxyphenyl)e thylene, as a white crystal, was obtained (about 80% yield).

MS(m/Z)=512.33. $^1$HNMR(ppm)δ: 7.08(d,1H,2'-H);6.92(d,1H,6'-H);6.76(d,1H,5'-H);6.62(s,2H,2,6'-H);6.49(d,1H,J=12.2Hz,1aH);6.43(d,1H,J=12.2Hz,1a'-H);4.18(q,2H,-$CH_2$);3.86(s,3H,4-$OCH_3$);3.70(s,6H,3,5-$OCH_3$);2.74(t,2H,-$CH_2$);2.36(dt,4H,-$NCH_2$-);2.33(t,2H,-$COCH_2$);1.40(m,4H,-$CH_2$-);1.35(m,4H,-$CH_2$-); 1.33(t,3H,-$CH_3$);0.96(t,6H,-$CH_3$).

### Example 6

Preparation of (*Z*)-1-(3,4,5-trimethoxyphenyl)-2-((3-(bis(2-*n*-butyl)amino)propionamide)-4-methoxyphenyl) ethylene

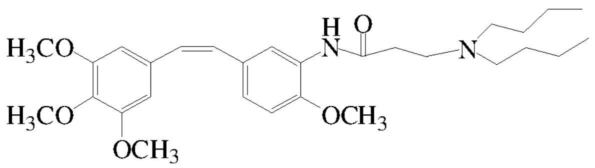

Step 1: (*Z*)-1-(3,4,5-trimethoxyphenyl)-2-(3-bromopropionamide)-4-methoxyphenyl)ethylene was synthesized according to the method in Example 3.

Step 2: (*Z*)-1-(3,4,5-trimethoxyphenyl)-2-((3-(bis(2-*n*-propyl)amino)propionamide)-4-methoxyphenyl )ethylene was synthesized according to the method in Example 1. MS(m/Z)=498.31. $^1$HNMR(ppm)δ: 7.08(d,1H,2'-H);6.92(d,1H,6'-H);6.76(d,1H,5'-H);6.62(s,2H,2,6'-H);6.49(d,1H,J=12.2Hz,1aH);6.43(d,1H,J=12.2Hz,1a'-H);3.86(s,3H,4-$OCH_3$);3.73(s,3H,-$OCH_3$);3.70(s,6H,3,5-$OCH_3$);2 .74(t,2H,-$CH_2$);2.36(dt,4H,-$NCH_2$-);2.33(t,2H,-$COCH_2$);1.40(m,4H,-$CH_2$-);1.33(m,4H,-$CH_2$-) ;0.96(t,6H,-$CH_3$).

### Example 7

Preparation of (*Z*)-1-(3,4,5-trimethoxyphenyl)-2-((3-(bis(2-*n*-propyl)amino)propionamide)-4-trifluoroethoxy phenyl)ethylene

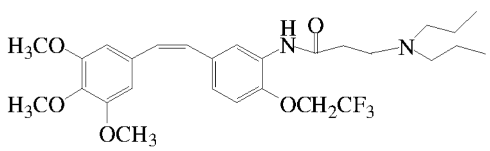

Step 1: (*Z*)-1-(3,4,5-trimethoxyphenyl)-2-(3-amino-4-trifluoroethoxyphenyl)ethylene was synthesized according

to the method in Example 1.

Step 2: (*Z*)-1-(3,4,5-trimethoxyphenyl)-2-((3-(bis(2-*n*-propyl)amino)propionamide)-4-trifluoroethoxy phenyl)ethylene was synthesized according to the method in Example 1. MS(m/Z)=538.27. $^1$HNMR(ppm)δ: 7.08(d,1H,2'-H);6.92(d,1H,6'-H);6.76(d,1H,5'-H);6.62(s,2H,2,6'-H);6.49(d,1H,J=12.2Hz,1aH);6.43(d,1H,J=12.2Hz,1a'-H);4.46(d,2H,-$OCH_2CF_3$);3.86(s,9H,3,4,5-$OCH_3$);2.74(t,2H,-$CH_2$ );2.36(dt,4H,-$NCH_2$-);2.33(t,2H,-$COCH_2$);1.40(m,4H,-$CH_2$-);0.96(t,6H,-$CH_3$).

**Example 8**

Preparation of (*Z*)-1-(3,4,5-trimethoxyphenyl)-2-((3-(bis(2-*n*-propyl)amino)propionamide)-4-difluoromethox yphenyl)ethylene

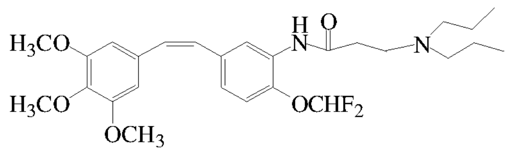

Step 1: (*Z*)-1-(3,4,5-trimethoxyphenyl)-2-(3-amino-4-difluoromethoxyphenyl)ethylene was synthesized according to the method in Example 3.

Step 2: (*Z*)-1-(3,4,5-trimethoxyphenyl)-2-((3-(bis(2-*n*-propyl)amino)propionamide)-4-difluoromethox yphenyl)ethylene was synthesized according to the method in Example 1. MS(m/Z)=506.26. $^1$HNMR(ppm)δ: 7.36(d,1H,-$CHF_2$);7.08(d,1H,2'-H);6.92(d,1H,6'-H);6.76(d,1H,5'-H);6.62(s,2H,2,6'-H);6.49( d,1H,J=12.2Hz,1a-H);6.43(d,1H,J=12.2Hz,1a'-H);3.73(s,9H,3,4,5-$OCH_3$);2.74(t,2H,-$CH_2$);2. 36(dt,4H,-$NCH_2$-);2.33(t,2H,-$COCH_2$);1.43(m,4H,-$CH_2$-);0.96(t,6H,-$CH_3$).

**Example 9**

Preparation of (*Z*)-1-(3,4,5-trimethoxyphenyl)-2-((3-(bis(2-*n*-butyl)amino)propionamide)-4-trifluoroethoxyp henyl)ethylene

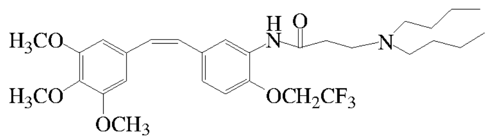

Step 1: (Z)-1-(3,4,5-trimethoxyphenyl)-2-(3-amino-4-trifluoroethoxyphenyl)ethylene was synthesized according to the method in Example 1.

Step 2: (*Z*)-1-(3,4,5-trimethoxyphenyl)-2-((3-(bis(2-*n*-butyl)amino)propionamide)-4-trifluoroethoxyp henyl)ethylene was synthesized according to the method in Example 1. MS(m/Z)=566.30. $^1$HNMR(ppm)δ: 7.08(d,1H,2'-H);6.92(d,1H,6'-H);6.76(d,1H,5'-H);6.62(s,2H,2,6'-H);6.49(d,1H,J=12.2Hz,1aH);6.43(d,1H,J=12.2Hz,1a'-H);4.46(d,2H,-$OCH_2CF_3$);3.86(s,9H,3,4,5-$OCH_3$);2.74(t,2H,-$CH_2$ );2.36(dt,4H,-$NCH_2$-);2.33(t,2H,-$COCH_2$);1.40(m,4H,-$CH_2$-);1.33(m,4H,-$CH_2$-);0.96(t,6H,-C $H_3$).

**Example 10**

Preparation of (*Z*)-1-(3,4,5-trimethoxyphenyl)-2-((3-(bis(2-*n*-butyl)amino)propionamide)-4-difluoromethoxy phenyl)ethylene

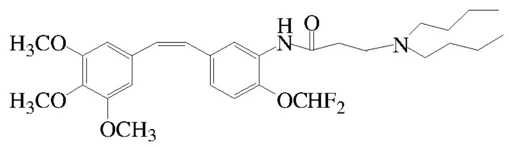

Step 1: (Z)-1-(3,4,5-trimethoxyphenyl)-2-(3-amino-4-difluoromethoxyphenyl)ethylene was synthesized according to the method in Example 3.

Step 2: (*Z*)-1-(3,4,5-trimethoxyphenyl)-2-((3-(bis(2-*n*-butyl)amino)propionamide)-4-difluoromethoxy phenyl)ethylene was synthesized according to the method in Example 1. MS(m/Z)=534.29. $^1$HNMR(ppm)δ: 7.36(d,1H,-$CHF_2$);7.08(d,1H,2'-H);6.92(d,1H,6'-H);6.76(d,1H,5'-H);6.62(s,2H,2,6'-H);6.49( d,1H,J=12.2Hz,1a-H);6.43(d,1H,J=12.2Hz,1a'-H);3.73(s,9H,3,4,5-$OCH_3$);2.74(t,2H,-$CH_2$);2. 36(dt,4H,-$NCH_2$-);2.33(t,2H,-$COCH_2$);1.39(m,4H,-$CH_2$-);1.33(m,4H,-$CH_2$-);0.96(t,6H,-$CH_3$).

## Example 11

Preparation of (*Z*)-1-(3,4,5-trimethoxyphenyl)-2-((3-(bis(2-hydroxyethyl)amino)propionamide)-4-ethoxyphe nyl)ethylene

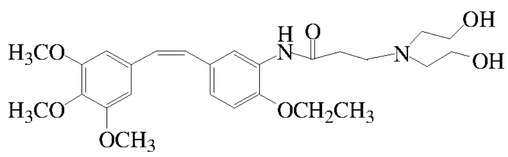

Step 1: (Z)-1-(3,4,5-trimethoxyphenyl)-2-(3-amino-4-ethoxyphenyl)ethylene was synthesized according to the method in Example 1.

Step 2: (*Z*)-1-(3,4,5-trimethoxyphenyl)-2-((3-(bis(2-hydroxyethyl)amino)propionamide)-4-ethoxyphe nyl)ethylene was synthesized according to the method in Example 1. MS(m/Z)=488.25. $^1$HNMR(ppm)δ: 7.68(d,1H,2'-H);6.92(d,1H,6'-H);6.76(d,1H,5'-H);6.62(s,2H,2,6'-H);6.49(d,1H,J=12.2Hz,1aH);6.43(d,1H,J=12.2Hz,1a'-H);3.98(-$OCH_2$-);3.86(s,3H,4-$OCH_3$);3.70(s,6H,3,5-$OCH_3$);3.63( q,4H,-$CH_2OH$);2.74(q,2H,-$CH_2N$-);2.55(dt,4H,-$NCH_2$-);2.33(q,2H,-$COCH_2$-);1.33(q,3H,-$CH_3$ ).

## Example 12

Preparation of (*Z*)-1-(3,4,5-trimethoxyphenyl)-2-((3-(bis(2-hydroxyethyl)amino)propionamide)-4-methoxyp henyl)ethylene

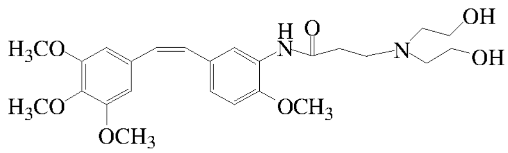

Step 1: (*Z*)-1-(3,4,5-trimethoxyphenyl)-2-(3-amino-4-methoxyphenyl)ethylene was synthesized according to the method in Example 3.

Step 2: (*Z*)-1-(3,4,5-trimethoxyphenyl)-2-((3-(bis(2-hydroxyethyl)amino)propionamide)-4-methoxyp henyl)ethylene was synthesized according to the method in Example 1. MS(m/Z)=474.24. $^1$HNMR(ppm)δ: 7.68(d,1H,2'-H);6.92(d,1H,6'-H);6.76(d,1H,5'-H);6.62(s,2H,2,6'-H);6.49(d,1H,J=12.2Hz,1aH);6.43(d,1H,J=12.2Hz,1a'-H);3.86(s,3H,4-$OCH_3$);3.70(s,9H,3,3',5-$OCH_3$);3.63(q,4H,-$CH_2O$ H);2.74(q,2H,-$CH_2N$-);2.55(dt,4H,-$NCH_2$-);2.33(q,2H,-$COCH_2$-).

## Example 13

Preparation of (*Z*)-1-(3,4,5-trimethoxyphenyl)-2-((3-(2,2-difluoroethylamino)propionamide)-4-ethoxyphenyl )ethylene

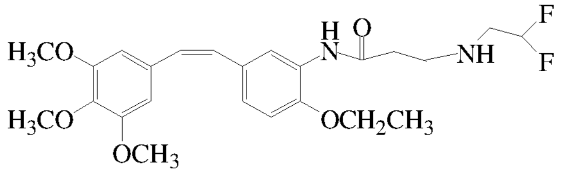

Step 1: (Z)-1-(3,4,5-trimethoxyphenyl)-2-(3-amino-4-ethoxyphenyl)ethylene was synthesized according to the method in Example 1.

Step 2: (*Z*)-1-(3,4,5-trimethoxyphenyl)-2-((3-(2,2-difluoroethylamino)propionamide)-4-ethoxyphenyl )ethylene was synthesized according to the method in Example 1. MS(m/Z)=464.21. $^1$HNMR(ppm)δ: 7.68(d,1H,2'-H);6.92(d,1H,6'-H);6.76(d,1H,5'-H);6.62(s,2H,2,6'-H);6.49(d,1H,J=12.2Hz,1aH);6.43(d,1H,J=12.2Hz,1a'-H);5.39(t,1H,-$CHF_2$);3.98(q,2H,-$OCH_2$);3.86(s,3H,4-$OCH_3$);3.70 (s,6H,3,5-$OCH_3$);2.93(m,2H,-$CH_2$-);2.91(t,2H,-$NCH_2$-);2.35(t,2H,-$COCH_2$);1.33(t,3H,-$CH_3$).

**Example 14**

Preparation of (*Z*)-1-(3,4,5-trimethoxyphenyl)-2-((3-(2,2-dimethoxyethylamino)propionamide)-4-ethoxyphe nyl)ethylene

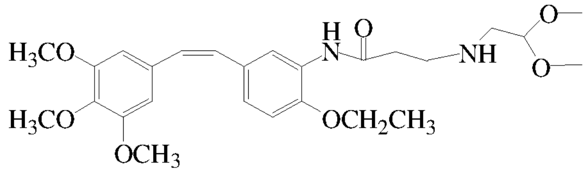

Step 1: (Z)-1-(3,4,5-trimethoxyphenyl)-2-(3-amino-4-ethoxyphenyl)ethylene was synthesized according to the method in Example 1.

Step 2: (*Z*)-1-(3,4,5-trimethoxyphenyl)-2-((3-(2,2-dimethoxyethylamino)propionamide)-4-ethoxyphe nyl)ethylene was synthesized according to the method in Example 1. MS(m/Z)=488.25. $^1$HNMR(ppm)δ: 7.68(d,1H,2'-H);6.92(d,1H,6'-H);6.76(d,1H,5'-H);6.62(s,2H,2,6'-H);6.49(d,1H,J=12.2Hz,1aH);6.43(d,1H,J=12.2Hz,1a'-H);4.43(t,1H,-CH-);3.98(q,2H,-$OCH_2$-);3.86(s,3H,4-$OCH_3$);3.70( s,6H,3,5-$OCH_3$);3.24(s,6H,-$OCH_3$);2.93(t,2H,-$NCH_2$-);2.85(t,2H,-$CH_2$-);2.35(t,2H,-$COCH_2$); 1.33(t,3H,-$CH_3$).

**Example 15. Pharmaceutical formulation**

The present invention provides several pharmaceutical composition formulations for diseases associated with abnormal neovascularization, including oral formulations such as tablets, capsules and the like. The following "active compounds" are the compound of general formula (I), the pharmaceutically acceptable salts thereof, the hydrates thereof, the solvates thereof, the solvates of the pharmaceutically acceptable salts or the isomers thereof disclosed herein.

| (a) Tablet I | Unit (mg) |
|---|---|
| Active compound | 100 |
| Lactose | 188.75 |
| Microcrystalline cellulose | 100 |
| Croscarmellose sodium | 6 |
| Starch | 2.25 |
| Magnesium stearate | 3 |

| (b) Tablet II | Unit (mg) |
|---|---|
| Active compound | 50 |
| Lactose | 218 |
| Croscarmellose sodium | 15 |
| Starch | 15 |
| Polyvinylpyrrolidone | 2 |
| Magnesium stearate | 3 |

| (c) Capsule | Unit (mg) |
|---|---|
| Active compound | 50 |
| Lactose | 218 |
| Magnesium stearate | 3 |

**Example 16. Acute toxicity study in animals**

Male ICR mice weighed 23 ± 2 g, clean grade, were selected. The route of administration was intragastric (ig) administration, which is consistent with the intended oral route for clinical use.

Mice were randomly divided into 8 groups by weight: the blank control group, Example 1 group, Example 5 group, Example 11 group, Example 12 group, Example 13 group, Example 14 group and control group. Among these, the control group received amino-combretastatin serinamide hydrochloride as the control (hereinafter referred to as control).

Preparation of the compounds used in the example groups: The compounds obtained in Example 1, Example 3, Example 4, Example 5, Example 6, Example 7, Example 8, Example 9, Example 10, Example 11, Example 12, Example 13 and Example 14 were prepared into hydrochloride salts according to the method disclosed in Example 2.

Preparation of control: The control was prepared with starting material aminoethoxy combretastatin (i.e., the product obtained in Step 2 of Example 1 of the present invention) through the following procedures:

(1) Aminoethoxy combretastatin, Fmoc-serine, DCC and HOBt were dissolved in DMF. The reaction mixture was stirred at room temperature for 5 h for reaction, which was monitored by TLC. After the reaction was completed, the mixture was cooled. Ethyl acetate was added for dilution, and the resultant mixture was well mixed, filtered, dried over anhydrous magnesium sulfate, concentrated at reduced pressure and separated by flash column chromatography to obtain a white foam substance.

(2) (*Z*)-1-(3,4,5-trimethoxyphenyl)-2-(3-amino-4-ethoxyphenyl)ethylene-Fmoc-serinamide obtained above was dissolved in a mixed solvent of methanol and dichloromethane. A 2 N sodium hydroxide solution was added with stirring, and the system was reacted at room temperature for 24 h. The reaction was monitored by TLC. After the reaction was completed, the mixture was cooled. A saturated sodium chloride solution was added, and the resultant mixture was well mixed and extracted with dichloromethane 3 times. The organic phase was dried over anhydrous magnesium sulfate, concentrated at reduced pressure and separated by column chromatography at normal pressure to obtain a colorless foam substance.

Pre-treatment: 48 mice were deprived of food but not water for 12 h before the study, and randomized into groups of 10 by body weight. The treatment groups were intragastrically administered once at 0.25 mL/10 g body weight, and the water control group received an equivalent amount of distilled water through intragastric administration. The $LD_{100}$ value (the amount for 100% death), $LD_0$ (the amount for 0 death) and the corresponding range r value between the dose groups were calculated, and $LD_{50}$ was determined.

Study procedures: 160 mice were randomized into 8 groups of 20 mice by body weight. After deprivation of food but not water for 12 h before the study, each group was intragastrically given the compound once at 0.3 mL/10 g body weight. Since no death was observed after 12 h, the second dose was given. The control group received equivalent volumes of water. The daily dose of each group was calculated; the control group received equal volumes of water and was subjected to an acute toxicity study for observation and the body weight of the mice was recorded daily for 14 days. By calculation, the results for oral $LD_{50}$ of each compound are shown in Table 1:

Table 1:

| Compound | LD50 (mg/Kg) |
|---|---|
| Hydrochloride of Example 1 | 1200 |
| Hydrochloride of Example 3 | 1000 |
| Hydrochloride of Example 4 | 1100 |
| Hydrochloride of Example 5 | 1200 |
| Hydrochloride of Example 6 | 900 |
| Hydrochloride of Example 7 | 1200 |
| Hydrochloride of Example 8 | 1100 |
| Hydrochloride of Example 9 | 1000 |
| Hydrochloride of Example 10 | 900 |
| Hydrochloride of Example 11 | 1100 |
| Hydrochloride of Example 12 | 1100 |

(continued)

| Compound | LD50 (mg/Kg) |
|---|---|
| Hydrochloride of Example 13 | 1000 |
| Hydrochloride of Example 14 | 1000 |
| Control | 200 |

The result showed that after the amino side chain of amino-combretastatin was modified, the LD50 in mice was increased by 5-6 folds, indicating that the compounds have significantly reduced acute toxicity and significantly improved safety for clinical use.

**Example 17. Efficacy study in nude mouse with xenograft tumor**

BALB/cA-nude mice aged 6 to 7 weeks were purchased from Shanghai Slac Laboratory Animal Co., Ltd. The nude mice were subcutaneously grafted with human liver cancer cells Bel-7402, colon cancer cells HT-29, gastric cancer cells SGC-7901 or non-small cell lung cancer cells A549. After the tumor grew to 100 to 250 $mm^3$, the animals were randomized (d0). The tumor volumes and body weights were measured twice or thrice a week, and the data were recorded. Tumor volume (V) was calculated as follows:

$$V = 1/2 \times a \times b^2$$

$$T/C\ (\%) = (T - T_0)\ /\ (C - C_0) \times 100$$

wherein a and b represent length and width, respectively; T and C are the tumor volumes at the end of study; T0 and C0 are the tumor volumes at the start of study.

All samples were diluted to the desired concentration with 50% PEG400 in distilled water. The regimen was once daily for 21 days at 50 mg/kg mouse body weight through intragastric administration, and 50 mg/kg for the control. The compounds obtained in Examples 1, 3, 4, 5, 6, 7, 8, 9, 10, 11, 12, 13 and 14 and the control were prepared into corresponding hydrochloride as the dosing compounds according to the method disclosed in Example 2 to investigate their efficacy on human liver cancer cell Bel-7402, colon cancer cell HT-29, gastric cancer cell SGC-7901 or non-small cell lung cancer cell A549 xenograft tumors in nude mice and to compare the compounds with the control (prepared as in Example 16). The results are shown in Table 2.

Table 2:

| Sources | % tumor inhibition (D21) | | | |
|---|---|---|---|---|
| | Bel-7402 | HT-29 | SGC-7901 | A549 |
| Example 1 | 92 | 93 | 82 | 69 |
| Example 3 | 89 | 88 | 78 | 65 |
| Example 4 | 90 | 91 | 76 | 67 |
| Example 5 | 88 | 82 | 80 | 63 |
| Example 6 | 89 | 90 | 81 | 62 |
| Example 7 | 81 | 79 | 65 | 66 |
| Example 8 | 90 | 88 | 71 | 67 |
| Example 9 | 79 | 85 | 80 | 61 |
| Example 10 | 86 | 81 | 73 | 60 |
| Example 11 | 88 | 89 | 65 | 63 |
| Example 12 | 79 | 79 | 77 | 58 |
| Example 13 | 82 | 77 | 72 | 63 |

(continued)

| Sources | % tumor inhibition (D21) | | | |
|---|---|---|---|---|
| | Bel-7402 | HT-29 | SGC-7901 | A549 |
| Example 14 | 82 | 71 | 80 | 66 |
| Control | 39 | 42 | 34 | 30 |

Conclusion: The compounds can significantly inhibit the growth of human liver cancer cell Bel-7402, colon cancer cell HT-29, gastric cancer cell SGC-7901 and non-small cell lung cancer cell A549 xenograft tumors in nude mice through intragastric administration, with significantly improved inhibition rates as compared with that of the control.

Described above are specific embodiments of the present invention. It should be appreciated that those of ordinary skills in the art can also make several improvements and modifications without departing from the principle of the present invention, and such improvements and modifications shall fall within the protection scope of the present invention.

## Claims

1. A compound of general formula (I), a pharmaceutically acceptable salt thereof, a hydrate thereof, a solvate thereof, a solvate of the pharmaceutically acceptable salt and an isomer thereof, wherein the general formula (I) is:

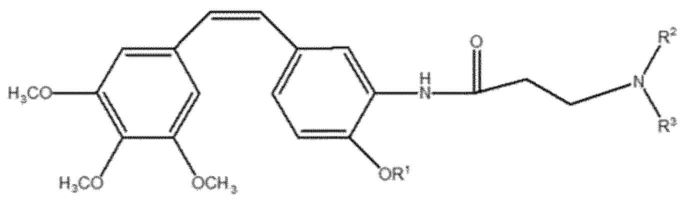

wherein $R^1$ is selected from a C1-C3 alkyl and a C1-C3 haloalkyl;
$R^2$ and $R^3$ are each independently selected from a C1-C6 alkyl, a C1-C6 haloalkyl and a C1-C6 alcohol group.

2. The compound of general formula (I), the pharmaceutically acceptable salt thereof, the hydrate thereof, the solvate thereof, the solvate of the pharmaceutically acceptable salt and the isomer thereof according to claim 1, wherein:

$R^1$ is selected from methyl, ethyl, difluoromethyl and trifluoroethyl;
$R^2$ and $R^3$ are each independently selected from methyl, ethyl, n-propyl, isopropyl, n-butyl, n-pentyl, n-hexyl, propenyl and propynyl; more preferably, $R^2 = R^3$; even more preferably, $R^2$ and $R^3$ are simultaneously selected from one of methyl, ethyl, n-propyl, isopropyl, n-butyl, n-pentyl, n-hexyl, propenyl and propynyl.

3. The compound of general formula (I), the pharmaceutically acceptable salt thereof, the hydrate thereof, the solvate thereof, the solvate of the pharmaceutically acceptable salt and the isomer thereof according to claim 1, wherein the compound is:

(*Z*)-1-(3,4,5-trimethoxyphenyl)-2-((3-(bis(2-*n*-propyl)amino)propionamide)-4-ethoxyphenyl)e thylene,
(*Z*)-1-(3,4,5-trimethoxyphenyl)-2-((3-(bis(2-*n*-propyl)amino)propionamide)-4-methoxyphenyl )ethylene,
(*Z*)-1-(3,4,5-trimethoxyphenyl)-2-((3-(bis(2-*n*-butyl)amino)propionamide)-4-ethoxyphenyl)et hylene,
(*Z*)-1-(3,4,5-trimethoxyphenyl)-2-((3-(bis(2-*n*-butyl)amino)propionamide)-4-methoxyphenyl) ethylene,
(*Z*)-1-(3,4,5-trimethoxyphenyl)-2-((3-(bis(2-ethyl)amino)propionamide)-4-ethoxyphenyl)ethy lene,
(*Z*)-1-(3,4,5-trimethoxyphenyl)-2-((3-(bis(2-*n*-propyl)amino)propionamide)-4-trifluoroethoxy phenyl),
(*Z*)-1-(3,4,5-trimethoxyphenyl)-2-((3-(bis(2-*n*-propyl)amino)propionamide)-4-difluoromethox yphenyl)ethylene,
(*Z*)-1-(3,4,5-trimethoxyphenyl)-2-((3-(bis(2-n-butyl)amino)propionamide)-4-trifluoroethoxyp henyl)ethylene,
(*Z*)-1-(3,4,5-trimethoxyphenyl)-2-((3-(bis(2-*n*-butyl)amino)propionamide)-4-difluoromethoxy phenyl)ethylene,
(*Z*)-1-(3,4,5-trimethoxyphenyl)-2-((3-(bis(2-hydroxyethyl)amino)propionamide)-4-ethoxyphe nyl)ethylene,
(*Z*)-1-(3,4,5-trimethoxyphenyl)-2-((3-(bis(2-hydroxyethyl)amino)propionamide)-4-methoxyp henyl)ethylene,
(*Z*)-1-(3,4,5-trimethoxyphenyl)-2-((3-(2,2-difluoroethylamino)propionamide)-4-ethoxyphenyl )ethylene or

(*Z*)-1-(3,4,5-trimethoxyphenyl)-2-((3-(2,2-dimethoxyethylamino)propionamide)-4-ethoxyphe nyl)ethylene.

4. The compound of general formula (I), the pharmaceutically acceptable salt thereof, the hydrate thereof, the solvate thereof, the solvate of the pharmaceutically acceptable salt and the isomer thereof according to claim 1, wherein the pharmaceutically acceptable salt refers to a salt formed by one or more basic salt-forming groups and an acid, wherein the acid is an organic acid and/or an inorganic acid.

5. A medicament or pharmaceutical composition prepared from the compound of general formula (I), the pharmaceutically acceptable salt thereof, the hydrate thereof, the solvate thereof, the solvate of the pharmaceutically acceptable salt or the isomer thereof according to any one of claims 1 to 4.

6. The medicament or pharmaceutical composition according to claim 5, wherein the medicament or pharmaceutical composition is in a formulation for topical, enteral or parenteral administration.

7. The medicament or pharmaceutical composition according to claim 6, wherein the medicament or pharmaceutical composition prepared from the compound of general formula (I), the pharmaceutically acceptable salt thereof, the hydrate thereof, the solvate thereof, the solvate of the pharmaceutically acceptable salt and the isomer thereof is a tablet or a capsule.

8. Use of the compound of general formula (I), the pharmaceutically acceptable salt thereof, the hydrate thereof, the solvate thereof, the solvate of the pharmaceutically acceptable salt and the isomer thereof according to any one of claims 1 to 4 in preparing a medicament for treating a disease caused by abnormal neovascularization.

9. Use of the compound of general formula (I), the pharmaceutically acceptable salt thereof, the hydrate thereof, the solvate thereof, the solvate of the pharmaceutically acceptable salt and the isomer thereof according to any one of claims 1 to 4 in preparing a tubulin aggregation inhibitor.

**INTERNATIONAL SEARCH REPORT**

International application No.
**PCT/CN2021/128275**

**A. CLASSIFICATION OF SUBJECT MATTER**

C07C 237/08(2006.01)i; C07C 231/12(2006.01)i; C07C 231/02(2006.01)i; C07C 233/25(2006.01)i; C07C 213/02(2006.01)i; C07C 217/02(2006.01)i; C07C 201/12(2006.01)i; C07C 205/37(2006.01)i; C07C 41/22(2006.01)i; C07C 43/225(2006.01)i; C07F 9/54(2006.01)i; A61K 31/661(2006.01)i; A61K 31/167(2006.01)i; A61P 35/00(2006.01)i; A61P 35/02(2006.01)i; A61P 35/02(2006.01)i

According to International Patent Classification (IPC) or to both national classification and IPC

**B. FIELDS SEARCHED**

Minimum documentation searched (classification system followed by classification symbols)

C07C,A61K.A61P

Documentation searched other than minimum documentation to the extent that such documents are included in the fields searched

Electronic data base consulted during the international search (name of data base and, where practicable, search terms used)

CNABS, DWPI, CNKI, STN(REG, CAPLUS): 华耀, 康普立停, 康布瑞汀 , 康普瑞汀 , 康普瑞丁 , 康布他汀 , 康普瑞塔卡汀, Combretastatin , CA4P

**C. DOCUMENTS CONSIDERED TO BE RELEVANT**

| Category* | Citation of document, with indication, where appropriate, of the relevant passages | Relevant to claim No. |
|---|---|---|
| A | EP 2942345 A1 (COUNCIL SCIENT IND. RES) 11 November 2015 (2015-11-11)<br>claims 1-7 | 1-9 |
| A | CN 103012248 A (ZHEJIANG DADE PHARMACEUTICAL CO., LTD.) 03 April 2013 (2013-04-03)<br>claims 1-11 | 1-9 |
| A | CN 107382796 A (SHANGHAI INSTITUTE OF TECHNOLOGY; SHANGHAI ECUST BIOMEDICINE CO., LTD.) 24 November 2017 (2017-11-24)<br>claims 1-7 | 1-9 |
| PX | CN 112225673 A (YIWU HUAYAO PHARMACEUTICAL TECHNOLOGY CO., LTD.) 15 January 2021 (2021-01-15)<br>claims 1-9 | 1-9 |
| A | CN 101085743 A (ZHEJIANG DADE PHARMACEUTICAL GROUP CO., LTD.) 12 December 2007 (2007-12-12)<br>claims 1-10 | 1-9 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 December 2021** | **30 January 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

INTERNATIONAL SEARCH REPORT

International application No.
PCT/CN2021/128275

C. DOCUMENTS CONSIDERED TO BE RELEVANT

| Category* | Citation of document, with indication, where appropriate, of the relevant passages | Relevant to claim No. |
|---|---|---|
| A | CN 103539642 A (SHANGHAI INSTITUTE OF TECHNOLOGY) 29 January 2014 (2014-01-29) see entire document | 1-9 |
| A | WO 2016067311 A1 (COUNCIL SCIENT IND. RES) 06 May 2016 (2016-05-06) claims 1-5 | 1-9 |
| A | CN 101139358 A (ZHEJIANG DADE PHARMACEUTICAL CO., LTD.) 12 March 2008 (2008-03-12) claims 1-10 | 1-9 |
| A | CN 101220054 A (CHENGDU HENGJI MEDICAL TECHNOLOGY CO., LTD.) 16 July 2008 (2008-07-16) see entire document | 1-9 |
| A | CN 104447598 A (ZHEJIANG DADE PHARMACEUTICAL CO., LTD.) 25 March 2015 (2015-03-25) see entire document | 1-9 |
| A | CN 101074189 A (YONG, Zhiquan; XU, Xiaoping) 21 November 2007 (2007-11-21) see entire document | 1-9 |

Form PCT/ISA/210 (second sheet) (January 2015)

**INTERNATIONAL SEARCH REPORT**
**Information on patent family members**

International application No.
**PCT/CN2021/128275**

| Patent document cited in search report | Publication date (day/month/year) | Patent family member(s) | Publication date (day/month/year) |
|---|---|---|---|
| EP 2942345 A1 | 11 November 2015 | US 2015322009 A1 | 12 November 2015 |
| | | US 9487482 B2 | 08 November 2016 |
| | | EP 2942345 B1 | 28 March 2018 |
| CN 103012248 A | 03 April 2013 | CN 103012248 B | 05 November 2014 |
| | | WO 2014108066 A1 | 17 July 2014 |
| CN 107382796 A | 24 November 2017 | None | |
| CN 112225673 A | 15 January 2021 | None | |
| CN 101085743 A | 12 December 2007 | KR 20090023465 A | 04 March 2009 |
| | | KR 101321960 B1 | 25 October 2013 |
| | | ES 2363716 T3 | 12 August 2011 |
| | | AT 501999 T | 15 April 2011 |
| | | CA 2660760 A1 | 13 December 2007 |
| | | CA 2660760 C | 08 January 2013 |
| | | WO 2007140662 A1 | 13 December 2007 |
| | | DE 602006020787 D1 | 28 April 2011 |
| | | JP 2009539779 A | 19 November 2009 |
| | | JP 5100749 B2 | 19 December 2012 |
| | | DK 2025661 T3 | 14 June 2011 |
| | | CN 101085743 B | 15 February 2012 |
| | | AU 2006344313 A1 | 13 December 2007 |
| | | AU 2006344313 B2 | 26 July 2012 |
| | | AU 2006344313 B8 | 06 December 2012 |
| | | RU 2008153041 A | 20 July 2010 |
| | | RU 2417216 C2 | 27 April 2011 |
| | | IN 255273 A1 | 08 February 2013 |
| | | US 2008306027 A1 | 11 December 2008 |
| | | US 7786098 B2 | 31 August 2010 |
| | | EP 2025661 A1 | 18 February 2009 |
| | | EP 2025661 A4 | 28 July 2010 |
| | | EP 2025661 B1 | 16 March 2011 |
| | | IN 2792MUMNP2008 A | 20 February 2009 |
| CN 103539642 A | 29 January 2014 | None | |
| WO 2016067311 A1 | 06 May 2016 | JP 2017533894 A | 16 November 2017 |
| | | JP 6854753 B2 | 07 April 2021 |
| | | WO 2016067311 A4 | 22 September 2016 |
| | | US 2017327462 A1 | 16 November 2017 |
| | | US 10246411 B2 | 02 April 2019 |
| | | EP 3212613 A1 | 06 September 2017 |
| | | EP 3212613 B1 | 16 December 2020 |
| CN 101139358 A | 12 March 2008 | KR 20090048504 A | 13 May 2009 |
| | | KR 101349925 B1 | 14 January 2014 |
| | | AU 2007295835 A1 | 20 March 2008 |
| | | AU 2007295835 B2 | 12 July 2012 |
| | | IN 1542DELNP2009 A | 29 May 2009 |
| | | WO 2008031333 A1 | 20 March 2008 |
| | | EP 2065358 A1 | 03 June 2009 |
| | | EP 2065358 A4 | 25 November 2009 |
| | | EP 2065358 B1 | 08 July 2015 |
| | | CA 2662737 A1 | 20 March 2008 |

Form PCT/ISA/210 (patent family annex) (January 2015)

**INTERNATIONAL SEARCH REPORT**
**Information on patent family members**

International application No.
**PCT/CN2021/128275**

| Patent document cited in search report | Publication date (day/month/year) | Patent family member(s) | Publication date (day/month/year) |
|---|---|---|---|
| | | CA 2662737 C | 11 June 2013 |
| | | JP 2010502656 A | 28 January 2010 |
| | | JP 5250876 B2 | 31 July 2013 |
| | | RU 2009117488 A | 10 November 2010 |
| | | RU 2451664 C2 | 27 May 2012 |
| | | US 2009170956 A1 | 02 July 2009 |
| | | CN 101139358 B | 12 October 2011 |
| CN 101220054 A | 16 July 2008 | CN 101220054 B | 25 May 2011 |
| CN 104447598 A | 25 March 2015 | None | |
| CN 101074189 A | 21 November 2007 | CN 101074189 B | 13 April 2011 |

Form PCT/ISA/210 (patent family annex) (January 2015)